(19) Europäisches Patentamt
European Patent Office
Office européen des brevets

(11) Publication number: **0 165 215**
**B1**

(12) **EUROPEAN PATENT SPECIFICATION**

(45) Date of publication of the patent specification: **19.04.89**

(51) Int. Cl.[4]: **C 07 C 15/04,** C 07 C 15/085, C 07 C 6/12, C 07 C 4/18, B 01 J 29/34

(21) Application number: **85830143.5**

(22) Date of filling: **12.06.85**

(54) **Method for the production of benzene and cumene.**

(30) Priority: **15.06.84 IT 2142984**

(43) Date of publication of application: **18.12.85 Bulletin 85/51**

(45) Publication of the grant of the patent: **19.04.89 Bulletin 89/16**

(84) Designated Contracting States: **AT BE CH DE FR GB LI LU NL SE**

(56) References cited:
**DE-A-2 127 481**
**GB-A-896 390**

(73) Proprietor: **ENICHEM ANIC S.p.A., Via Ruggero Settimo 55, I-90139 Palermo (IT)**

(72) Inventor: **Messina, Giuseppe, Via Perpignan, 27, I-07041 Alghero (Sassari) (IT)**
Inventor: **Moretti, Mario Domenico, Via Oriani, 5, I-07100 Sassari (IT)**
Inventor: **Brundu, Gavino, Via Amficore, 70, I-07046 Porto Torres (Sassari) (IT)**

(74) Representative: **Rambelli, Paolo, Jacobacci- Casetta & Perani S.p.A. Via Alfieri 17, I-10121 Torino (IT)**

**EP 0 165 215 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

## Description

The present invention relates to a method for the production of benzene and cumene from the heavy by-products of the process for the production of cumene by the catalytic alkylation of benzene with propylene.

It is known in the art to produce cumene by the catalytic alkylation of benzene with propylene, for example on supported phosphoric-acid catalysts.

In these processes, in addition to the cumene (isopropylbenzene), by-products are also formed which result from the further alkylation of the benzene, in particular di-isopropylbenzenes and products of the alkylation of benzene with higher olefins such as butenes, pentenes, hexenes (dimers of propylene), present in the technical propylene or which form during the alkylation reaction.

These heavy by-products, which will be termed cumene bottoms or simply bottoms in the present specification, separate at the bottom of the cumene distillation column and result in a smaller yield of the useful reaction product and loss of valued compounds.

It has thus been proposed to subject the di-isopropylbenzenes in the cumene bottoms to transalkylation by the addition of benzene to the bottoms, the reaction being carried out in the presence of catalysts such as boron and aluminium halides commonly known as Friedel Crafts catalysts. The use of such catalysts, however, involves various disadvantages due to corrosion and ecological problems as well as problems resulting from the high consumption of the catalysts themselves.

In order to overcome these disadvantages, it has been proposed in the art to use heterogeneous transalkylation catalysts such as, for example, zeolites (USP-3 385 906) which are active in a temperature range of from 130 to 250° C, or zeolites containing a metal, such as nickel, palladium or platinum within their structure (DE-OS 2 127 481) which are active in a hydrogen environment and within a temperature range of from 275 to 350° C.

The present invention is based on the finding that catalysts constituted by natural silico-aluminates with a silica alumina ratio greater than or equal to 3/1, pretreated with a mineral acid, and on which a hydrogenating metal has been deposited, are active not only in the transalkylation of dialkylbenzenes (like the catalysts of the prior art) but also in the dealkylation of monoalkylbenzenes, when the reaction is carried out in a hydrogen environment, in accordance with the following general reactions:

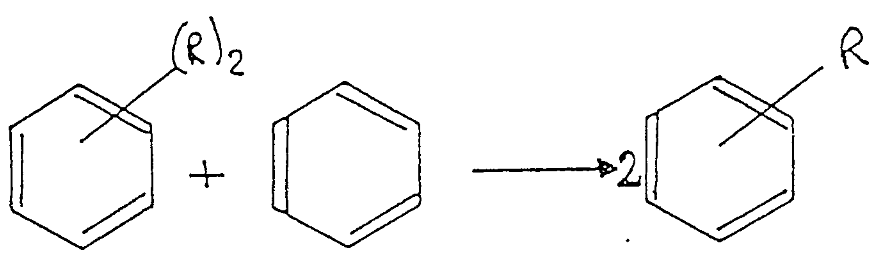

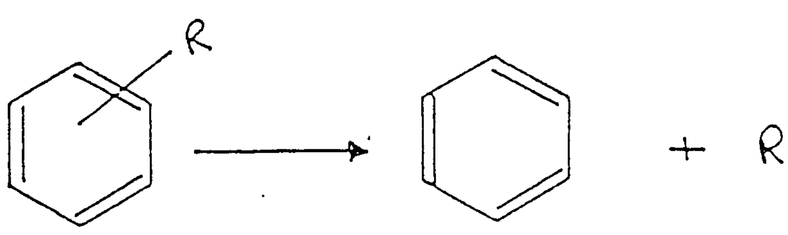

Consequently, some of the benzene required by the transalkylation reaction is generated within the reaction environment itself, with obvious economic advantages. In particular the consumption of benzene is of the order of 0.15 - 0.20 parts by weight per part by weight of cumene produced, compared with the stoichiometric quantity required by the transalkylation reaction which is 0.65 parts by weight of benzene per part by weight of cumene.

In accordance with this, the present invention relates to a method for the dealkylation and the transalkylation of cumene bottoms by mixing them with benzene and by treating the mixture in liquid phase under a pressure of hydrogen of from 10 to 60 bar in the presence of a catalyst constituted by a natural silico-aluminate with a silica/alumina ratio greater or equal to 3 : 1, pretreated with a mineral acid, characterised by the fact that the reaction is runned at a temperature of from 200 to 270°C and that the natural silico-aluminate catalyst is impregnated with an aqueous solution of a metal belonging to group VIII of the periodic system of the elements, then dried at 100 - 130°C, calcined at from 200 to 400°C and finally activated in an atmosphere of hydrogen for 3 to 5 hours at from 150 to 500° C.

Natural silico-aluminates suitable for the purpose are bentonites, particularly those having a high montmorillonite content. It is known that montmorillonite is a sodium, calcium and magnesium silico-aluminate

having the following general formula:

$[Al_{1.67}Mg_{0.33}Na_{0.33}]\ Si_4O_{10}\,(OH)_2$.

The percentage montmorillonite in bentonites generally varies from 60 to 80 % by weight, the remainder being constituted by materials such as silica, calcite, illite. The treatment of the bentonites with mineral acids, such as sulphuric, hydrofluoric, hydrochloric acid, partly or completely removes the alkali metals, which are replaced by hydrogen, and one thus obtains the so-called activated acid earths.

The acid earth commercially known under the trademark FILTROL made by the Filtrol Company, Los Angeles (USA), is, of the commercial acid earths, that most particularly suitable for the purposes of the present invention.

These activated acid earths themselves have transalkylating catalytic activities as described in GB-A-896 390 however under the conditions of the present inventive method, they rapidly become deactivated and are thus not useful for this purpose. Therefore, according to the present invention, a metal from group VIII of the periodic system, generally palladium, platinum, nickel or cobalt, is deposited on the acid earth.

It has been found, surprisingly, that these catalysts do not have an appreciable hydrogenating action on the aromatic nucleus under the conditions of the present inventive method and give conversions of the cumene bottoms of from 50 to 100 % with a selectivity towards cumene of more than about 90 %.

Among all the catalytic metals nickel has been found to be the most suitable because it has practically no hydrogenating effect and it has a very high resistance to ageing.

The quantity of metal in the catalyst may vary from 0.05 to 5 % by weight with respect to the silico-aluminate of the support, with preferred values of from 0.1 to 1 % in the case of palladium or platinum and from 1 to 5 % by weight in the case of nickel or cobalt.

The catalyst may be prepared by impregnating the activated acid earth with an aqueous, possibly acidic solution of a salt of the metal which it is intended to deposit (for example palladium chloride for nickel nitrate). The acid earth thus treated is then dried at a temperature of the order of 100 - 130°C and finally calcined at temperatures of the order of 200 - 400°C.

The catalyst obtained is conveniently treated before use in an atmosphere of hydrogen for 3 to 5 hours at a temperature of from 150 to 500°C.

In an embodiment of the method of the present invention the cumene bottoms as such, or with an addition of benzene, are fed to and brought into contact with the catalyst.

In particular, the molar ratio between the added benzene and the bottoms may vary generally from 0/1 to 20/1. It is however noted that the presence of added benzene favours the transalkylation and dealkylation reactions and retards the ageing of the catalyst.

On the other hand it is noted that a large quantity of added benzene does not give substantial advantages. Hence in the preferred embodiment, a mixture of benzene and cumene bottoms is fed to the catalyst in a molar ratio of 2/1 to 10/1.

The preferred reaction temperatures are within the range 200 to 300°C. The hydrogen pressures may vary in general from 10 to 60 bar and, in a preferred embodiment, are within the range 20 to 40 bar.

In practice this corresponds roughly to a hydrogen/organic molar ratio of 1/1 to 10/1 for the wider pressure range and from 2/1 to 5/1 for the preferred pressure range.

In practice the reaction may be carried out by passing the liquid reaction mixture over the catalyst deposited in the form of a fixed bed, at a rate of passage of the mixture of from 0.5 to 5 volumes of the mixture per volume of catalyst per hour.

The reaction products obtained, after separation of the hydrogen, are treated to separate the valued constituents, particularly benzene and cumene. In a preferred embodiment, the reaction products are conveyed to the distillation reaction stage of the plant for the production of cumene by the alkylation of benzene with propylene.

If the activity of the catalyst tends to fall during use, the supply of cumene bottoms may be suspended and the catalyst may be treated with benzene alone in the presence of hydrogen, in other conditions appropriate for the reaction. Thus complete activation of the catalyst is achieved in a period of the order of 0.5 - 3 hours.

The method of the present invention is particularly advantageous when used in combination with any method for the production of cumene by the catalytic alkylation of benzene with propylene.

In these alkylation processes, carried out in the liquid or gaseous phases, at a temperature within the range 50 - 400°C, benzene and propylene are conveyed to the catalyst (phosphoric acid, aluminium halide, silicaalumina, acid resins ), with a molar excess of the first over the second, and the propylene is converted substantially completely to give a reaction product constituted by the excess benzene, cumene and cumene bottoms.

These latter consist essentially of di-isopropylbenzenes (about 70 %) and products of the alkylation of benzene with oligomers of propylene (about 25 %) and constitute the feed for the process of the present invention once separated from the benzene and the cumene.

The experimental examples which follow are illustrative. In the Examples, the parts and percentages are expressed by weight if not otherwise indicated.

**Example 1**

The commercial acid earth FILTROL G 24, having the following composition

| | |
|---|---|
| $SiO_2$ | 67.2 % |
| $Al_2O_3$ | 13.8 % |
| $Fe_2O_3$ | 0.99 % |
| Ca | 0.82 % |
| Na | 0.02 % |
| $H_2O$ | balance to 100, |

in the form of 30 to 60 mesh granules, is added to a solution of palladium chloride ($Pd\ Cl_2$) dissolved in 6N hydrochloric acid, the mass being kept under agitation. The quantity of palladium chloride is such as to ensure a 0.1 % by weight of palladium metal in the finished catalyst. The mixture is then kept under agitation at 80°C until the greater part of the hydrochloric acid has evaporated.

It is then dried in an oven at 110°C for 4 hours and subsequently in a muffle at 240°C for 4 hours.

The catalyst obtained is sieved to remove powder with a grain size of less than 60 mesh and is then loaded into a cylindrical reactor (height 20 cm; diameter 2.5 cm) having a thermostatic jacket.

The catalyst is activated in the reactor by heat in a flow of hydrogen (30 l/hour) at a pressure of 35 ban, at a temperature of 240°C for 5 hours.

In this example cumene bottoms are used which result from a process for the alkylation of benzene with propylene on a supported phosphoric-acid catalyst, and which have the following composition:

| | |
|---|---|
| cumene | 1.13 % |
| butylbenzenes | 3.96 % |
| pentylbenzenes | 4.46 % |
| meta-di-isopropylbenzene | 23.23 % |
| ortho-di-isopropylbenzene | 8.3 % |
| para-di-isopropylbenzene | 25.92 % |
| hexylbenzenes | 22.9 % |
| other alkylaromatics | 10.1 % |

These cumene bottoms are distilled to separate the fraction having the following composition:

| | |
|---|---|
| cumene | 1.28 % |
| butylbenzenes | 4.46 % |
| pentylbenzenes | 5.02 % |
| meta-di-isopropylbenzene | 25.27 % |
| ortho-di-isopropylbenzene | 9.5 % |
| para-di-isopropylbenzene | 27.74 % |
| hexylbenzenes | 26.74 % |

The fraction distilled is mixed with various quantities of benzene to give the mixtures A, B and C which are treated by the method of the present invention.

| | Mixture | | |
|---|---|---|---|
| | A | B | C |
| benzene | 45.41 % | 64.49 % | 67.06 % |
| cumene | 0.5 % | 0.30 % | 0.24 % |
| butylbenzenes | 2.07 % | 1.31 % | 1.14 % |
| pentylbenzenes | 2.18 % | 1.48 % | 1.20 % |
| meta-di-isopropylbenzene | 14.12 % | 9.19 % | 8.60 % |
| ortho-di-isopropylbenzene | 5.30 % | 3.45 % | 3.32 % |
| para-di-isopropylbenzene | 15.48 % | 10.08 % | 9.44 % |
| hexylbenzenes | 19.94 % | 9.71 % | 9.10 % |

The mixture A has a benzene/bottoms molar ratio of 1.89/1, and a bromine number of 0.6 and is fed continuously to the catalyst described above and treated under the conditions given in Table 1 below. The duration of the test is 130 hours. Table 1 also gives the compositions of the reaction mixture for average samples taken at the times indicated and the values of the molar conversion of the cumene bottoms, the percentage weight ratio of the cumene produced to the bottoms converted and the bromine number of the reaction products.

**Table 1**

| | | | | | |
|---|---|---|---|---|---|
| Time (hours) | | 1-35 | 36-70 | 71-105 | 106-130 |
| Benzene/bottoms molar ratio | | 1.89 | 1.89 | 1.89 | 1.89 |
| Hydrogen/organic molar ratio | | 2.87 | 2.87 | 2.87 | 2.87 |
| Temperature (°C) | | 200 | 200 | 200 | 200 |
| Pressure (bar) | | 34 | 34 | 34 | 34 |
| Rate (volumes/volume hour) | | 1.33 | 1.33 | 1.33 | 1.33 |
| Composition samples | | | | | |
| Paraffins + naphthenes | (%) | 7.86 | 5.49 | 5.22 | 5.04 |
| benzene | (%) | 49.75 | 50.34 | 50.36 | 49.74 |
| cumene | (%) | 11.44 | 9.46 | 8.28 | 7.7 |
| butylbenzenes | (%) | 0.75 | 1.00 | 1.28 | 1.05 |
| pentylbenzenes | (%) | 0.26 | 0.42 | 0.73 | 0.81 |
| di-isopropylbenzenes + hexylbenzenes | (%) | 26.99 | 30.14 | 30.51 | 32.3 |
| heavies | (%) | 3.00 | 3.15 | 3.62 | 3.35 |
| Bottoms molar conversion | (%) | 45.84 | 39.51 | 38.78 | 35.20 |
| (cumene produced/bottoms converted)100 | | 50.1 | 48.0 | 42.8 | 43.9 |
| Bromine number | | 0.01 | 0.01 | 0.01 | 0.01 |

Similarly, the mixture B, which has a benzene/bottoms molar ratio of 4.13/1 and a bromine number of 0.5, is treated on the catalyst used in the preceding test, over a period of from 131 to 182 hours. The data from the test are given in Table 2.

**Table 2**

| | | | |
|---|---|---|---|
| Time (hours) | | 131-170 | 171-182 |
| Benzene/bottoms molar ratio | | 4.13 | 4.13 |
| Hydrogen/organic molar ratio | | 2.5 | 2.5 |
| Temperature (°C) | | 200 | 220 |
| Pressure (bar) | | 34 | 34 |
| Rate (volumes/volume hour) | | 1.33 | 1.33 |
| Composition samples | | | |
| Paraffins + naphthenes | (%) | 3.83 | 4.94 |
| benzene | (%) | 68.52 | 65.64 |
| cumene | (%) | 6.40 | 11.40 |
| butylbenzenes | (%) | 0.58 | 0.51 |
| pentylbenzenes | (%) | 0.32 | 0.34 |
| di-isopropylbenzenes + hexylbenzenes | (%) | 19.07 | 16.03 |
| heavies | (%) | 1.29 | 1.20 |
| Bottoms molar conversion | (%) | 41.19 | 50.55 |
| (cumene produced/bottoms converted)100 | | 47.9 | 69.1 |
| Bromine number | | 0.01 | 0.01 |

Similarly, the mixture C, which has a benzene/bottoms molar ratio of 4.59/1 and a bromine number of 0.5, is treated on the catalyst used in the preceding test, over a period of 183 to 320 hours. The data from the test are given in Table 3.

**Table 3**

| | | | | | | | |
|---|---|---|---|---|---|---|---|
| Time (hours) | | 183-220 | 221-264 | 265-280 | 281-290 | 291-301 | 302-320 |
| Benzene/bottoms molar ratio | | 4.59 | 4.59 | 4.59 | 4.59 | 4.59 | 4.59 |
| Hydrogen/organic molar ratio | | 2.5 | 2.5 | 2.5 | 5.20 | 1.25 | 2.5 |
| Temperature (°C) | | 220 | 220 | 220 | 220 | 220 | 220 |
| Pressure (bar) | | 34 | 34 | 34 | 34 | 34 | 34 |
| Rate (volumes/volume hour) | | 1.33 | 1.33 | 1.33 | 1.33 | 1.33 | 1.33 |
| Composition samples | | | | | | | |
| Paraffins + naphthenes | (%) | 3.89 | 4.1 | 4.24 | 4.41 | 3.47 | 3.92 |
| benzene | (%) | 70.04 | 67.9 | 69.51 | 75.31 | 67.01 | 70.2 |
| cumene | (%) | 9.21 | 10.48 | 9.95 | 11.11 | 9.96 | 9.05 |
| butylbenzenes | (%) | 0.55 | 0.65 | 0.64 | 0.28 | 0.60 | 0.62 |
| pentylbenzenes | (%) | 0.38 | 0.66 | 0.64 | 0.31 | 0.77 | 0.76 |
| di-isopropylbenzenes + hexylbenzenes | (%) | 14.88 | 14.69 | 13.4 | 8.33 | 16.69 | 13.96 |
| heavies | (%) | 1.06 | 3.16 | 1.62 | 0.26 | 1.49 | 1.40 |

| | | | | | | |
|---|---|---|---|---|---|---|
| Bottoms molar conversion (%) | 51.00 | 51.63 | 55.88 | 72.57 | 45.04 | 54.03 |
| (cumene produced/bottoms converted)100 | 59.5 | 66.8 | 58.6 | 50.4 | 72.8 | 55.1 |
| Bromine number | 0.01 | 0.01 | 0.01 | 0.01 | 0.01 | 0.01 |

**Example 2**

25 % of the weight of the catalyst discharged from the reactor at the end of the tests of Example 1 is replaced by the same fresh catalyst.

The catalytic composition thus obtained is used to treat the following mixtures.

To cumene bottoms having the following composition:

| | |
|---|---|
| cumene | 0.65 % |
| butylbenzenes | 3.91 % |
| pentylbenzenes | 5.35 % |
| meta-di-isopropylbenzene | 22.61 % |
| ortho-di-isopropylbenzene | 10.05 % |
| para-di-isopropylbenzene | 31.50 % |
| hexylbenzenes | 21.39 % |
| other alkylaromatics | 4.54 % |

there is added benzene to give the mixture F having the following composition:

| | |
|---|---|
| benzene | 81.00 % |
| cumene | 0.12 % |
| butylbenzenes | 0.74 % |
| pentylbenzenes | 1.02 % |
| meta-di-isopropylbenzene | 4.30 % |
| ortho-di-isopropylbenzene | 1.91 % |
| para-di-isopropylbenzene | 5.99 % |
| hexylbenzenes | 4.06 % |
| other alkylaromatics | 0.86 % |

The mixture F has a benzene/bottoms molar ratio of 9.64/1 and a bromine number of 0.25.

Moreover cumene bottoms, the composition whereof is given above, are distilled to separate the fraction having the following composition:

| | |
|---|---|
| cumene | 0.83 % |
| butylbenzenes | 4.97 % |
| pentylbenzenes | 6.80 % |
| meta-di-isopropylbenzene | 25.54 % |
| ortho-di-isopropylbenzene | 10.96 % |
| para-di-isopropylbenzene | 29.08 % |
| hexylbenzenes | 21.86 |

This distilled fraction is mixed with benzene to give mixtures D and E having the compositions:

**Mixture D:**

| | |
|---|---|
| benzene | 67.25 % |
| cumene | 0.27 % |
| butylbenzenes | 1.63 |
| pentylbenzenes | 2.23 |
| meta-di-isopropylbenzene | 8.36 |
| ortho-di-isopropylbenzene | 3.59 |
| para-di-isopropylbenzene | 9.52 |
| hexylbenzenes | 7.16 |

**Mixture E:**

| | |
|---|---|
| benzene | 80.4 % |
| cumene | 0.16 % |
| butylbenzenes | 0.97 % |
| pentylbenzenes | 1.33 % |
| meta-di-isopropylbenzene | 5.01 % |

| | |
|---|---|
| ortho-di-isopropylbenzene | 2.15 % |
| para-di-isopropylbenzene | 5.70 % |
| hexylbenzenes | 4.28 % |

The mixture D has a benzene/bottoms molar ratio of 4.87/1 and a bromine number of 0.5 and the mixture E has a benzene/bottoms molar ratio of 10.35/1 and a bromine number of 0.25.

Other cumene bottoms having the following composition:

| | |
|---|---|
| cumene | 0.12 % |
| butylbenzenes | 1.76 % |
| pentylbenzenes | 5.34 % |
| meta-di-isopropylbenzene | 24.32 % |
| ortho-di-isopropylbenzene | 11.08 % |
| para-di-isopropylbenzene | 30.19 % |
| hexylbenzenes | 21.86 % |
| other alkylaromatics | 5.33 % |

are distilled to separate the fraction having the following composition:

| | |
|---|---|
| cumene | 0.14 % |
| butylbenzenes | 1.99 % |
| bentylbenzenes | 6.02 % |
| meta-di-isopropylbenzene | 25.32 % |
| ortho-di-isopropylbenzene | 12.05 % |
| para-di-isopropylbenzene | 31.38 % |
| hexylbenzenes | 22.92 % |
| other alkylaromatics | 0.10 % |

The distilled fraction is mixed with benzene to give the mixture G having the following composition:

| | |
|---|---|
| benzene | 80.96 % |
| cumene | 0.03 % |
| butylbenzenes | 0.38 % |
| pentylbenzenes | 1.15 % |
| meta-di-isopropylbenzene | 4.82 % |
| ortho-di-isopropylbenzene | 9.29 % |
| para-di-isopropylbenzene | 5.97 % |
| hexylbenzenes | 4.36 % |

The mixture G has a benzene/bottoms molar ratio of 9.64/1 and a bromine number of 0.26.

The mixture D is used in the test for 1 to 112 hours (Table 4). The mixture E is used in the test from 113 to 171 hours (Table 5). The mixture F is used in the test from 172 to 308 hours (Table 7).

The mixture G is used in the test from 309 to 350 hours (Table 6).

**Table 4**

| | | | | | |
|---|---|---|---|---|---|
| Time (hours) | | 1-16 | 17-51 | 52 - 82 | 83 -112 |
| Benzene/bottoms molar ratio | | 4.87 | 4.87 | 4.87 | 4.87 |
| Hydrogen/organic molar ratio | | 2.5 | 2.5 | 2.5 | 2.5 |
| Temperature (°C) | | 220 | 220 | 220 | 250 |
| Pressure (bar) | | 35 | 35 | 25 | 35 |
| Rate (volumes/volume.hour) | | 1.67 | 1.67 | 1.67 | 1.67 |
| Composition samples | | | | | |
| Paraffins + naphthenes | (%) | 5.22 | 4.32 | 4.28 | 6.01 |
| benzene | (%) | 71.98 | 70.76 | 72.17 | 63.12 |
| cumene | (%) | 19.47 | 18.04 | 11.81 | 25.50 |
| butylbenzenes | (%) | 0.37 | 0.41 | 0.37 | 0.56 |
| pentylbenzenes | (%) | 0.16 | 0.48 | 0.40 | 0.27 |
| di-isopropylbenzenes + hexylbenzenes | (%) | 8.75 | 11.25 | 10.09 | 4.14 |
| heavies | (%) | 0.05 | 0.73 | 0.79 | 0.29 |
| Bottoms conversion | (%) | 90.39 | 60.67 | 64.76 | 85.54 |
| (cumene produced/bottoms converted)100 | | 72.5 | 69.3 | 63.7 | 104.1 |
| Bromine number | | 0.01 | 0.01 | 0.01 | 0.01 |

**Table 5**

| | | | |
|---|---|---|---|
| Time (hours) | | 113 -152 | 153 -171 |
| Benzene/bottoms molar ratio | | 10.35 | 10.35 |
| Hydrogen/organic molar ratio | | 2.3 | 1.14 |
| Temperature (°C) | | 250 | 250 |
| Pressure (bar) | | 35 | 35 |
| Rate (volumes/volume-hour) | | 1.67 | 1.67 |
| Composition samples | | | |
| Paraffins + naphthenes | (%) | 3.90 | 3.74 |
| benzene | (%) | 76.04 | 79.28 |
| cumene | (%) | 17.89 | 15.54 |
| butylbenzenes | (%) | 0.37 | 0.28 |
| pentylbenzenes | (%) | 0.10 | 0.04 |
| di-isopropylbenzenes + hexylbenzenes | (%) | 1.70 | 1.09 |
| heavies | (%) | - | - |
| Bottoms molar conversion | (%) | 90.08 | 93.64 |
| (cumene produced/bottoms converted)100 | | 115.9 | 96.8 |
| Bromine number | | 0.01 | 0.01 |

**Table 6**

| | | |
|---|---|---|
| Time (hours) | | 309 - 350 |
| Benzene/bottoms molar ratio | | 9.64 |
| Hydrogen/organic molar ratio | | 2.28 |
| Temperature (°C) | | 270 |
| Pressure (bar) | | 35 |
| 1.67 (volumes/volume.hour) | | |
| Composition samples | | |
| Paraffins + naphthenes | (%) | 3.11 |
| benzene | (%) | 78.70 |
| cumene | (%) | 13.57 |
| butylbenzenes | (%) | 0.13 |
| pentylbenzenes | (%) | 0.20 |
| di-isopropylbenzenes + hexylbenzenes | (%) | 4.05 |
| heavies | (%) | 0.07 |
| Bottoms molar conversion | (%) | 76.55 |
| (cumene produced/bottoms converted)100 | | 101.7 |
| Bromine number | | 0.01 |

**Table 7**

| | | | | | |
|---|---|---|---|---|---|
| Time (hours) | | 172 - 216 | 217 - 243 | 244 - 280 | 281- 308 |
| Benzene/bottoms molar ratio | | 9.64 | 9.64 | 9.64 | 9.64 |
| Hydrogen/organic molar ratio | | 2.28 | 2.28 | 4.56 | 4.56 |
| Temperature (°C) | | 250 | 250 | 270 | 270 |
| Pressure (bar) | | 35 | 35 | 35 | 35 |
| Rate (volumes/volume hour) | | 1.67 | 1.67 | 1.67 | 1.67 |
| Composition samples | | | | | |
| Paraffins + naphthenes | (%) | 3.54 | 2.94 | 2.97 | 2.80 |
| benzene | (%) | 77.04 | 78.08 | 79.07 | 79.61 |
| cumene | (%) | 16.83 | 13.41 | 13.78 | 11.75 |
| butylbenzenes | (%) | 0.32 | 0.28 | 0.23 | 0.20 |
| pentylbenzenes | (%) | 0.08 | 0.22 | 0.18 | 0.22 |
| di-isopropylbenzenes + hexylbenzenes | (%) | 1.91 | 5.21 | 3.64 | 5.40 |
| heavies | (%) | 0.05 | 0.14 | 0.08 | 0.14 |
| Bottoms molar conversion | (%) | 88.25 | 87.96 | 77.61 | 66.79 |
| (cumene produced/bottoms converted)100 | | 117.3 | 121.3 | 109.2 | 108.2 |
| Bromine number | | 0.01 | 0.01 | 0.01 | 0.01 |

**Example 3**

An aqueous solution of nickel nitrate in distilled water was prepared. The commercial acid earth FILTROL G 24 described in Example 1 was added to this solution in quantities such as to give a nickel content (as the

metal) of 2 % in the finished catalyst. The addition was carried out with the solution under agitation, at 80° C. The greater part of the water was then evaporated and the solid obtained was dried at 100° C for 4 hours and at 350° C for 4 hours. The solid was sieved to remove fine powder and the catalyst was discharged into a jacketted tubular steel reactor (height 20 cm, diameter 2.5 cm) where it was activated by a flow of hydrogen (30 l/hour) at 350° C for 5 hours and at atmospheric pressure.

To the catalyst thus activated was fed the mixture H (1 to 10 hours) and the mixture I (103 to 270 hours) given in Example 2 above. The data are given in Tables 8 and 9 below.

**Table 8**

| | | | | | | |
|---|---|---|---|---|---|---|
| Time (hours) | | 1-10 | 11-26 | 27 - 54 | 55 -78 | 79 -102 |
| Benzene/bottoms molar ratio | | 3.64 | 3.64 | 3.64 | 3.64 | 3.64 |
| Hydrogen/organic molar ratio | | 1.40 | 1.40 | 1.40 | 1.40 | 1.40 |
| Temperature (° C) | | 250 | 250 | 250 | 250 | 250 |
| Pressure (bar) | | 35 | 35 | 35 | 35 | 35 |
| Rate (volumes/volume hour) | | 1.67 | 1.67 | 1.67 | 1.67 | 1.67 |
| Composition samples | | | | | | |
| Paraffins + naphthenes | (%) | 4.67 | 4.44 | 4.21 | 3.98 | 3.46 |
| benzene | (%) | 77.62 | 77.98 | 76.85 | 76.55 | 77.55 |
| cumene | (%) | 16.59 | 16.03 | 17.47 | 17.58 | 17.47 |
| butylbenzenes | (%) | 0.14 | 0.11 | 0.12 | 0.09 | 0.06 |
| pentylbenzenes | (%) | 0.20 | 0.29 | 0.29 | 0.30 | 0.23 |
| di-isopropylbenzenes + hexylbenzenes | (%) | 0.83 | 1.19 | 1.06 | 1.40 | 1.23 |
| heavies | (%) | - | - | - | - | - |
| Bottoms molar conversion | (%) | 95.3 | 93.2 | 93.9 | 92.0 | 92.9 |
| (cumene produced/bottoms converted)100 | | 99.9 | 98.7 | 106.7 | 109.6 | 107.8 |
| Bromine number | | 0.01 | 0.01 | 0.01 | 0.01 | 0.01 |

**Table 9**

| | | | | | | | |
|---|---|---|---|---|---|---|---|
| Time (hours) | | 103 -125 | 126 -147 | 148 -177 | 178 - 205 | 206 - 237 | 238 - 270 |
| Benzene/bottoms molar ratio | | 9.64 | 9.64 | 9.64 | 9.64 | 9.64 | 9.64 |
| Hydrogen/organic molar ratio | | 1.40 | 1.40 | 1.40 | 1.40 | 1.40 | 1.40 |
| Temperature (° C) | | 250 | 250 | 250 | 250 | 250 | 250 |
| Pressure (bar) | | 35 | 35 | 35 | 35 | 35 | 35 |
| Rate (volumes/volume hour) | | 1.67 | 1.67 | 1.67 | 1.67 | 1.67 | 1.67 |
| Composition samples | | | | | | | |
| Paraffins + naphthenes | (%) | 3.99 | 3.78 | 3.85 | 3.86 | 3.44 | 3.74 |
| benzene | (%) | 77.98 | 78.33 | 77.64 | 77.77 | 77.27 | 77.25 |
| cumene | (%) | 16.27 | 15.05 | 15.70 | 15.00 | 15.51 | 15.40 |
| butylbenzenes | (%) | 0.04 | 0.03 | 0.15 | 0.20 | 0.20 | 0.24 |
| pentylbenzenes | (%) | 0.25 | 0.31 | 0.16 | 0.23 | 0.23 | 0.16 |
| di-isopropylbenzenes + hexylbenzenes | (%) | 1.46 | 2.27 | 2.44 | 2.78 | 3.27 | 3.19 |
| heavies | (%) | 0.01 | 0.13 | 0.07 | 0.17 | 0.08 | - |
| Bottoms molar conversion | (%) | 91.0 | 86.0 | 85.0 | 83.0 | 79.9 | 80.4 |
| (cumene produced/bottoms converted)100 | | 109.9 | 107.6 | 113.6 | 111.3 | 119.4 | 117.8 |
| Bromine number | | 0.01 | 0.01 | 0.01 | 0.01 | 0.01 | 0.01 |

**Example 4**

The catalyst of Example 3 was used to treat the mixture L having the following composition:

| | |
|---|---|
| paraffins + naphthenes | 2.99 % |
| benzene | 78.35 % |
| cumene | 13.00 % |
| butylbenzenes | 0.09 % |
| pentylbenzenes | 0.24 % |
| meta-di-isopropylbenzene | 1.56 % |
| ortho-di-isopropylbenzene | 0.79 % |
| para-di-isopropylbenzene | 1.83 % |

| | |
|---|---|
| hexylbenzenes | 1.02 % |
| other alkylaromatics | 0.03 % |

This mixture, which has a bromine number of 0.40, is the liquid effluent from the cumene reactors and from the cumene bottoms.

The data from this test are given in Table 10 below.

**Table 10**

| | | |
|---|---|---|
| Time (hours) | | 1-25 |
| Hydrogen/organic molar ratio | | 1.4 |
| Temperature (°C) | | 250 |
| Pressure (bar) | | 35 |
| Rate (volumes/volume.hour) | | 1.67 |
| Composition samples | | |
| Paraffins + naphthenes | (%) | 5.11 |
| benzene | (%) | 78.07 |
| cumene | (%) | 16.81 |
| butylbenzenes | (%) | 0.06 |
| pentylbenzenes | (%) | - |
| meta-di-isopropylbenzene | (%) | 0.51 |
| ortho-di-isopropylbenzene | (%) | - |
| para-di-isopropylbenzene | (%) | 0.24 |
| hexylbenzenes | (%) | |
| other alkylaromatics | (%) | |
| Bottoms molar conversion | (%) | 82.1 |
| (cumene produced/bottoms converted)100 | | 85.6 |
| Bromine number | | 0.01 |

**Claims**

1. Method for the dealkylation and the transalkylation of cumene bottoms by mixing them with benzene and by treating the mixture in liquid phase under a pressure of hydrogen of from 10 to 60 bar in the presence of a catalyst constituted by a natural silico-aluminate with a silica/alumina ratio greater or equal to 3 : 1, pretreated with a mineral acid, characterised by the fact that the reaction is runned at a temperature of from 200 to 270°C and that the natural silico-aluminate catalyst is impregnated with an aqueous solution of a metal belonging to group VIII of the periodic system of the elements, then dried at 100 - 130°C, calcined at from 200 to 400°C and finally activated in an atmosphere of hydrogen for 3 to 5 hours at from 150 to 500°C.

2. Method according to Claim 1, characterised in that the catalyst contains a group VIII metal in quantities of from 0.05 to 5 % by weight with respect to the natural silico-aluminate.

3. Method according to Claim 2, characterised in that the catalyst contains palladium or platinum in quantities of from 0.1 to 1 % by weight with respect to the natural silico-aluminate.

4. Method according to Claim 2, characterised in that the catalyst contains nickel or cobalt in quantities of from 1 to 5 % by weight with respect to the natural silico-aluminate.

5. Method according to Claim 1, characterised in that it is carried out with a rate of passage of the liquid reaction mixture over the catalyst of from 0.5 to 5 volumes of the mixture itself per volume of catalyst per hour.

**Patentansprüche**

1. Verfahren zur Dealkylierung und Transalkylierung von Cumenrückständen durch Mischung dieser Rückstände mit Benzol und durch Behandlung dieser Mischung in flüssiger Phase unter einem Druck von Wasserstoff, von 10 bis 60 bar, in der Gegenwart eines Katalysators, der aus einem natürlichen, mit einer Mineralsäure vorbehandelten Silico-Aluminat mit einem Silico/Aluminiumoxid-Verhältnis größer oder gleich 3 : 1, besteht, dadurch gekennzeichnet, daß die Reaktion bei einer Temperatur zwischen 200 und 270°C durchgeführt wird, und daß der natürliche Silico-Aluminatkatalysator mit einer wäßrigen Lösung eines zur Gruppe VIII des periodischen Systems der Elemente gehörigen Metalls imprägniert, dann bei 100 bis 130°C getrocknet, bei 200 bis 400°C kalziniert und letztlich bei 150 bis 500°C in einer Wasserstoffatmosphäre 3 bis 5 Stunden aktiviert wird.

2. Verfahren nach Anspruch 1, dadurch gekennzeichnet, daß der Katalysator ein Metall der Gruppe VIII in einer Menge zwischen 0,05 bis 5 Gew.-% in Bezug auf das natürliche Silicoaluminat enthält.

3. Verfahren nach Anspruch 2, dadurch gekennzeichnet, daß der Katalysator Palladium oder Platin in einer Menge zwischen 0,1 bis 1 Gew.-% im Verhältnis auf das natürliche Silicoaluminat enthält.

4. Verfahren nach Anspruch 2, dadurch gekennzeichnet, daß der Katalysator Nickel oder Kobalt in einer Menge von 1 bis 5 Gew.-% in Bezug auf das natürliche Silicoaluminat enthält.

5. Verfahren nach Anspruch 1, dadurch gekennzeichnet, daß es mit einer Durchflußrate des flüssigen Reaktionsgemisches über den Katalysator von 0,5 bis 5 Volumseinheiten der Mischung selbst in Sezug auf das Volumen des Katalysators pro Stunde durchgeführt wird.

**Revendications**

1. Procédé pour la désalkylation et la transalkylation de queues de cumène en les mélangeant avec du benzène et en traitant le mélange en phase liquide sous une pression d'hydrogène de 10 à 60 bar en présence d'un catalyseur constitué par un silico-aluminate naturel ayant un rapport silice/alumine supérieur ou égal à 3 : 1, prétraité par un acide minéral, caractérisé en ce que la réaction est effectuée à une température de 200 à 270°C et en ce que le silico-aluminate naturel catalyseur est imprégné par une solution aqueuse d'un métal appartenant au groupe VIII de classification périodique des éléments, puis séché à 100 - 130°C, calciné de 200 à 400°C et enfin activé dans une atmosphère d'hydrogène pendant 3 à 5 h de 150 à 500°C.

2. Procédé selon la revendication 1, caractérisé en ce que le catalyseur contient un métal du groupe VIII en quantités de 0,05 à 5 % en poids par rapport au silico-aluminate naturel.

3. Procédé selon la revendication 2, caractérisé en ce que le catalyseur contient du palladium ou du platine en quantités de 0,1 à 1 % en poids par rapport au silico-aluminate naturel.

4. Procédé selon la revendication 2, caractérisé en ce que le catalyseur contient du nickel ou du cobalt en quantités de 1 à 5 % en poids par rapport au silico-aluminate naturel.

5. Procédé selon la revendication 1, caractérisé en ce qu'il est mis en oeuvre à une vitesse spatiale horaire du mélange de réaction liquide sur le catalyseur de 0,5 à 5 volumes du mélange lui-même par volume de catalyseur et par heure.